# EUROPÄISCHE PATENTSCHRIFT

(11) **EP 1 226 412 B1**
(45) Veröffentlichungstag und Bekanntmachung des Hinweises auf die Patenterteilung: **03.09.2003**
(21) Anmeldenummer: 00983040.7
(22) Anmeldetag: 19.10.2000
(51) Int. Cl.: G01J 5/34, A61B 5/00

(54) **TEMPERATURMESSEINRICHTUNG**
TEMPERATURE MEASURING DEVICE
DISPOSITIF DE MESURE DE TEMPERATURE

(30) Priorität: 20.10.1999 DE 19950516
(43) Veröffentlichungstag der Anmeldung: 31.07.2002
(73) Patentinhaber: Herbener, Heinz-Gerd, 52249 Eschweiler (DE)
(72) Erfinder: HERBENER, Heinz-Gerd, 52249 Eschweiler (DE); VOGELS, Nikolaus, 52072 Aachen (DE)
(74) Vertreter: Castell, Klaus, Dr.-Ing.
(86) Internationale Anmeldenummer: DE0003676
(87) Internationale Veröffentlichungsnummer: WO01029524

(56) Entgegenhaltungen:
- FR-A- 2 426 249
- GB-A- 2 170 228
- US-A- 3 333 103
- US-A- 3 533 399
- US-A- 3 847 139
- US-A- 4 015 591
- US-A- 4 190 058
- US-A- 4 524 778

## Beschreibung

Die Erfindung betrifft eine Temperaturmesseinrichtung mit einem z. B. auf die Haut eines Menschen oder Tieres aufbringbaren Flächenelement.

Temperaturmesseinrichtungen werden in verschiedensten Ausführungsvarianten vor allem für technische Messvorhaben verwendet. Sie dienen jedoch auch als sogenannte Fieberthermometer zur Messung der Körpertemperatur eines Menschen oder Tieres. Hierbei wird davon ausgegangen, dass die Oberflächentemperatur in bestimmten Hautbereichen mit der Körpertemperatur korreliert und somit durch Messung der Oberflächentemperatur beispielsweise im Achselbereich auf die Körpertemperatur zurückgeschlossen werden kann.

Derartige Temperaturmesseinrichtungen basieren zum Teil auf der Ausdehnung eines bestimmten Mediums wie Quecksilber oder einer alkoholischen Flüssigkeit. In neuerer Zeit werden diese Temperaturmesseinrichtungen jedoch mehr und mehr durch elektronische Messeinrichtungen ersetzt, die eine weit schnellere Ansprechzeit aufweisen.

Diese bekannten Messeinrichtungen sind zur punkluellen Temperaturmessung geeignet. Sie eignen sich jedoch nicht dazu, auf einer Fläche Zonen erhöhter Temperatur zu ermitteln. Hierzu dienen Einrichtungen zur Messung der Wärmestrahlung, die beispielsweise auch fotografisch aufgenommen werden kann. Diese Einrichtungen sind jedoch aufwendig in der Bedingung und kostspielig in der Anschaffung.

Zur Ermittlung von Krankheiten oder Krankheitsherden wäre es jedoch in vielen Fällen von Vorteil, wenn auf einer bestimmten Fläche des Körpers Bereiche erhöhter Temperatur festgestellt werden könnten. Diese Temperaturerhöhungen zeigen Regionen verstärkter Durchblutung an, die in vielen Fällen auf Entzündungen hinweisen. Zumindest ein Mediziner kann anhand von regionalen Temperaturerhöhungen auf mögliche Krankheiten zurückschließen oder feststellen, dass derartige Temperaturerhöhungen üblichen individuellen oder statistischen Schwankungen entsprechen.

Eine einfache Messeinrichtung zur Ermittlung regional erhöhter Körpertemperaturen würde somit neue medizinische Diagnoseverfahren ermöglichen und sie könnte auch im häuslichen Bereich die Messung der Hautoberflächentemperatur erleichtern.

Der Erfindung liegt daher die Aufgabe zugrunde, eine gatttnigsgemäße Temperaturmesseinrichtung derart weiterzubilden, dass sie besonders einfach anwendbar ist.

Diese Aufgabe wird dadurch gelöst, dass das Flächenelement einen Farbstoffträger enthält, der entweder bei Überschreiten oder bei Unterschreiten einer Grenztemperatur seine Farbe irreversibel ändert.

Dieser Farbstoffträger kann bei Erwärmung auf einen Wert oberhalb einer bestimmten Temperatur seine Farbe ändern und bei einem Abkühlen unter diese Temperatur die Farbe beibehalten. Er kann jedoch auch bei einer Abkühlung unter einen bestimmten Wert seine Farbe ändern und bei einer Erwärmung über diese oder eine andere bestimmte Temperatur seine Farbe beibehalten.

Farbpartikel, die bei einer bestimmten Temperatur ihre Farbe verändern und beim Abkühlen unter diese Temperatur ihre Farbe beibehalten sind bekannt. Erst durch das reversible Aufbringen derartiger Farbstoffträger mittels eines Flächenelementes beispielsweise auf die Haut eines Menschen oder Tieres werden diese Farbstoffträger zu einer Temperaturmesseinrichtung, die für verschiedenste Einsatzgebiete verwendet werden kann.

Diese Temperaturmesseinrichtungen können dazu verwendet werden, bei Lebensmitteln festzustellen, ob sie in einer geschlossenen Kühlkette gehalten wurden oder zwischenzeitlich zu hohen Temperaturen ausgesetzt waren. Beispielsweise könnte durch Aufbringen derartiger Messeinrichtungen festgestellt werden, ob Tiefkühlprodukte immer in einem Temperaturbereich zwischen -14° C und -18° C gelegen haben oder beispielsweise eine Temperatur von -10° überschritten haben. Entsprechende Temperaturbereiche bestehen für Kühlprodukte zwischen +5° C und +25° C oder für die Trockenlagerung bei +5° C bis +25° C. Heißprodukte sollten konstant bei 60° C bis 70° C gehalten werden und diesen Temperaturbereich nicht unterschreiten. Für Medikamente und Kosmetika bestehen vorgeschriebene Temperaturspannen zwischen +4° C und +25° C. Es ist somit im Interesse des Verbrauchers sicherzustellen, dass die Medikamente höheren oder niedrigeren Temperaturen nicht ausgesetzt waren. Dies kann durch eine erfindungsgemäße Temperaturmesseinrichtung überprüfen werden, die sowohl bei einem Unterschreiten der Temperatur von +4° C als auch bei einem Überschreiten der Temperatur von +25° C eine spezielle Färbung hervorruft, die vom Verbraucher beispielsweise auf der Verpackung erkennbar ist.

Auch technische Gase sollten während Transport und Lagerung in einer Temperaturspanne zwischen +15° C und +60° C gehalten werden, da sie sonst irreversibel geschädigt werden könnten oder zu hohe Drücke in den Druckgasflaschen entstehen. Beispielsweise ist bekannt, dass Ethylenoxid polymerisieren kann und sich dabei erhitzt. Eine polymerisierende Ethylenoxidflasche wird somit warm. Der Verbraucher hat verständlicherweise ein hohes Interesse, festzustellen, ob die Flasche schon einmal heiß geworden ist, um defekte Flaschen auszusortieren.

Da es eine Vielzahl an Produkten gibt, die bei Über- oder Unterschreiten einer kritischen Temperatur eine Veränderung erleiden, die sich meist auf die Produktqualität auswirkt, dienen die erfindungsgemäßen Temperaturmesseinrichtungen dazu, den Verbraucher oder den Anbieter auf derartige Schädigungen hinzuweisen.

Der Hinweis kann durch die bloße Verfärbung eines Flächenelementes erzielt werden. Vorteilhaft ist es jedoch, wenn das Flächenelement ein Schriftfeld ist. Dies führt dazu, dass bei Über- oder Unterschreiten des Temperaturgrenzwertes ein farbiges Schriftfeld erscheint, das zuvor noch nicht erkennbar war. Dies wird dadurch erzielt, dass auf dem Flächenelement Buchstaben oder andere Zeichen ihre Farbe ändern, sodass sie sich vom Hintergrund abheben. Verständlicherweise kann sich auch die Farbe des Hintergrunds ändern, sodass in der Farbe unveränderte Buchstaben oder Zeichen kontrastreich in Erscheinung treten.

Ein weiteres wichtiges Anwendungsgebiet der erfindungsgemäßen Temperaturmesseinrichtung ist das Aufbringen eines Flächenelementes auf die Haut eines Menschen oder Tieres. Hierbei ist vor allem auf die hohe Mortalitätsrate bei Brustkrebs hinzuweisen, die zu einem beachtlichen Teil auf eine unzureichende Früherkennung zurückzuführen ist.

Die bekannten Früherkennungsmaßnahmen beruhen auf dem Abtasten der Brust und der Durchleuchtung mittels Strahlung oder Ultraschall. Diese Verfahren setzen die unmittelbare Anwesenheit eines Fachmannes voraus und insbesondere bei Abtastverfahren ist fraglich, ob es möglicherweise auch das Brustkrebsentstehungsrisiko erhöht.

Eine Temperaturmessung mit der erfindungsgemäßen Temperaturmesseinrichtung über die gesamte Brustfläche kann hingegen regionale Temperaturerhöhungen aufzeigen, die einen definierten Grenzwert übersteigen. Sofern derartige Bereiche auftreten sollte im Rahmen einer ärztlichen Untersuchung die Ursache festgestellt werden, damit möglichst schnell Gegenmaßnahmen getroffen werden können.

Die Meßmethode eignet sich sowohl für den ärztlichen als auch für den häuslichen Bereich. Sie ist besonders einfach in der Durchführung und somit auch für den Laie anwendbar. Auch die notwendigen Materialien können preisgünstig hergestellt werden, sodass preisgünstige Vorsorgeuntersuchungen beispielsweise in einem 4 - Wochen - Rhythmus durchgeführt werden können. Dies erlaubt es auch Tendenzen zu ermitteln, um rechtzeitig den behandelnden Arzt aufzusuchen.

Die Temperaturmesseinrichtung eignet sich jedoch auch als einfaches Fieberthermometer. Hierbei wird der Farbstoffträger als Flächenelement beispielsweise auf die Haut eines Kleinkindes aufgetragen. Anhand der Farbentwicklung kann zumindest das Überschreiten einer vorher festgelegten Maximaltemperatur ermittelt werden, ab der ein ärztlicher Besuch oder eine genauere Messung anzuraten ist.

Vorteilhaft ist es, wenn der Farbstoffträger bei einer Temperatur zwischen 35 °C und 38 °C seine Farbe ändert. Beispielsweise bei Kindern geht man davon aus, dass ab einer mit einem Fieberthermometer gemessenen Temperatur von 37 °C bis 37,5 °C von Fieber gesprochen werden kann. Das heißt, hier liegt eine Grenztemperatur vor, bei deren Überschreitung beispielsweise ein ärztlicher Besuch anzuraten ist. Für dieses Anwendungsgebiet eignet sich somit ein Farbstoffträger, der bei einer Temperatur unter 37 °C eine Grünfarbung und darüber eine Rotfärbung aufweist.

Eine vorteilhafte Ausführungsform sieht vor, dass der Farbstoffträger bei Überschreiten verschiedener Grenztemperaturen jeweils seine Farbe ändert. Dies kann beispielsweise durch ein Gemisch an Farbstoffträgern erzielt werden, das mehrere verschiedene Farbstoffpartikel aufweist, die bei unterschiedlichen Temperaturen ihre Farbe ändern. Das Flächenelement erhält somit nach seiner Auflage beispielsweise auf die weibliche Brust ein Farbmuster, bei dem jede Farbe oder Farbmischung eine bestimmte Temperatur repräsentiert. Diese Farbverteilung kann fotografisch festgehalten werden, sodass die Farbvelteilung die Grundlage für eine spätere Analyse oder Untersuchung bilden kann.

In einer einfachen Ausführungsform ist das Flächenelement eine Farbschicht. Eine Farbschicht kann individuellen Formen optimal angepasst werden und es ist kein wesentlicher Druck notwendig, um die als Temperaturmesseinrichtung wirkende Farbschicht mit einer individuell geformten drei dimensionalen Fläche wie beispielsweise der Brust einer Frau in Verbindung zu bringen.

Die Farbschicht kann mit einem Pinsel oder Spachtel aufgetragen werden. Sofern die Farbschicht eine aufgesprühte Farbe ist, ist auf einfache Art und Weise eine gleichmäßige Verteilung zu erzielen.

Um die Farbe leicht entfernen zu können, wird vorgeschlagen, dass die Farbschicht abwaschbar ist. Die Farbe kann somit als Seife oder wie Seife aufgetragen werden, anschließend wird beobachtet, ob sich ein Farbumschlag einstellt, und nach einer vorbestimmten Zeit wird die Farbe wieder abgewaschen. Dieser Prozess ist einfach durchführbar und kann beliebig oft wiederholt werden.

Das Flächenelement kann auch eine hautverträgliche Creme oder Paste sein. Während eine Creme auch längere Zeit auf der Haut verbleiben kann, wird die Paste nach der Verwendung abgewaschen oder sie härtet aus, sodass sie auch abgepellt werden kann.

Vor allem bei einer aufgesprühten Farbschicht ist es vorteilhaft, wenn die Farbschicht einen entfernbaren Farbfilm bildet. Dieser Farbfilm ist nach der Temperaturmessung vorzugsweise einstückig entfernbar und bildet bei irreversibel verfärbten Farbpigmenten eine weiterverwendbare drei dimensionale Abbildung der vermessenen Körperpartie, mit der die Temperaturverteilung über die gemessene Fläche festgehalten werden kann.

Das Flächenelement kann ein geformtes oder formbares Element sein. Beispielsweise ein Tuch ist auf verschiedene Oberflächen auflegbar und nimmt die Form dieser Oberflächen an. Beispielsweise für die weibliche Brust können jedoch auch konvexgeformte Elemente verwendet werden, die den Körbchen eines Büstenhalters entsprechen. Diese Elemente verändern ihre Farbe entweder auf der der Haut zugewandten Seite oder sie sind so dünn bzw. temperaturleitfähig ausgebildet, dass die Verfärbung zumindest auch auf der der Haut abgewandten Seite erkennbar ist.

Eine Ausführungsform sieht vor, dass das Flächenelement Teil eines Büstenhalters ist. Dies erlaubt es, die Temperaturmesseinrichtung in einen Büstenhalter zu integrieren. Dadurch wird auf einfache Art und Weise eine regelmäßige Temperaturmessung erreicht.

Die Temperaturmesseinrichtung ist jedoch nicht nur für Messungen an der Haut eines Menschen oder Tieres geeignet sondern bietet sich auch für andere Flächenmessungen an. Nur beispielsweise wird auf die Vermessung der Temperaturentwicklung auf elektronischen Bauteilen, Platinen oder Computer-Chips verwiesen bei denen der Ort der stärksten Temperaturabgabe entweder besonders gekühlt werden muss oder auf eine schlechte Konstruktion hinweist. Die erfindungsgemäße Temperaturmesseinrichtung kann somit sowohl bei der Konstruktion von Hardware-Bauelementen als auch deren Betrieb helfen, regionale Temperaturerhöhungen zumindest ab einem bestimmten Grenzwert zu vermeiden.

Es wird daher ein elektronisches Bauteil vorgeschlagen, das eine erfindungsgemäße Temperaturmesseinrichtung aufweist. Außerdem wird ein Lebensmittel, ein Medikament, eine Kosmetik oder eine Überdruckgasflasche vorgeschlagen, die eine erfindungsgemäße Temperaturmesseinrichtung aufweisen.

Drei Ausführungsbeispiele erfindungsgemäßer Temperaturmesseinrichtungen sind in der Zeichnung dargestellt und werden im folgenden näher erläutert.

Es zeigt
- Figur 1: schematisch eine weibliche Brust mit aufgesprühter Farbschicht,
- Figur 2: schematisch einen Ausschnitt eines Büstenhalters mit einem eingearbeiteten verfärbbaren Flächenelement und
- Figur 3: einen Computerchip mit integrierter elektronischer Schaltung.

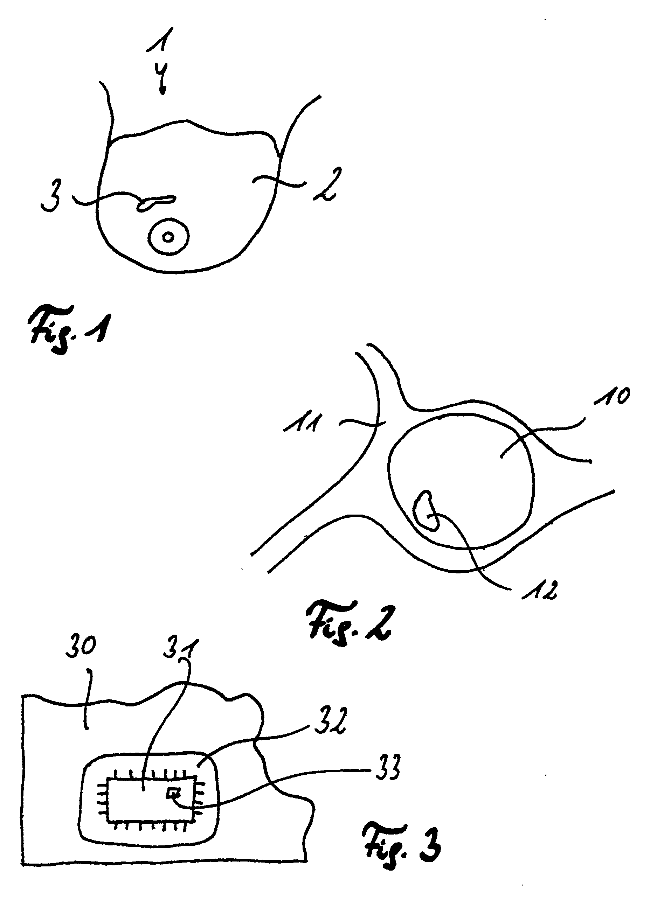

Die in Figur 1 schematisch dargestellte weibliche Brust 1 wurde hauchdünn mit einer Farbschicht 2 besprüht. Diese Farbschicht 2 bindet kurz nach dem Auftragen auf die menschliche Haut ab und bildet einen Farbfilm, der durch Farbstoffträger hellgrün gefärbt ist. Da der Farbfilm 2 extrem dünn ist, nimmt er sofort die Oberflächentemperatur der menschlichen Haut auf und die Farbstoffträger erhalten eine hellgrüne Farbe, die der Temperatur der Haut entspricht. In der Region 3 hat der Farbfilm eine bräunliche Verfärbung und dies weist daraufhin, dass in diesem Bereich eine erhöhte Oberflächentemperatur der Haut vorliegt. Dies ist in der Regel auf eine stärkere Durchblutung der darunterliegenden Körperpartie zurückzuführen. Eine derartige stärkere Durchblutung kann wiederum verschiedene Ursachen haben. Dies kann beispielsweise eine entzündete Milchdrüse, eine Druckstelle oder eine Gewebeanomalität wie beispielsweise ein Geschwür sein.

Der ausgehärtete Farbfilm kann nun von der Haut - vorzugsweise einstückig - entfernt werden. Er bildet eine Abbildung der Brust mit Verfärbungen in den Bereichen, in denen die Brust eine erhöhte Temperatur aufweist.

Die Farbstoffträger sind so ausgewählt, dass sie auch bei einem Abkühlen des Farbfilmes ihre Farbe nicht verlieren, sodass zumindest die braungefärbten Partien eingefärbt bleiben.

Der Farbfilm kann einem Frauenarzt zur genaueren Untersuchung vorgelegt werden und er kann aufbewahrt werden, um an in Zeitabständen nacheinander hergestellten Farbfilmen eine Veränderung der veifärbten Bereiche beobachten zu können. Eine Vergrößerung der Verfärbunysbereiche deutet auf eine Verschlimmerung der Erkrankung hin, während eine Verkleinerung eine Heilung nahe legt.

Die Figur 2 zeigt ein kreisrundes Wattepad 10, das in ein Körbchen eines Büstenhalters 11 eingelegt ist. Beim Tragen des Büstenhalters erwärmt sich das Wattepad 10 auf die Temperatur der weiblichen Brust und in Bereichen der weiblichen Brust, in denen eine erhöhte Temperatur festzustellen ist, wird auch das Wattepad stärker erwärmt. Diese Erwärmung führt bei Überschreitung einer Grenzteinperatur von 36 °C zu einem Farbumschlag von grün zu braun, sodass nach dem Tragen des Büstenhalters das Wattepad zur Diagnose entnommen werden kann. Eine spezielle Formgebung des Wattepads, beispielsweise als Dreieck, oder Farbmarkierungen ermöglichen ein Einlegen des Wattepads in einer bestimmten Ausrichtung in den Büstenhalter, sodass auch nach Entnahme des Wattepads eine Zuordnung bestimmter verfärbter Padbereiche 12 zu Oberflächenregionen der weiblichen Brust erleichtert wird.

Die Figur 3 zeigt eine in einem Eckbereich einer Platine 30 angeordnete integrierte Schaltung 31, die verschiedene Rechneraufgaben beim Betrieb des Rechners erledigt. Auf diese integrierte Schaltung 31 ist eine Folie 32 aufgeklebt, die verschiedene Farbpigmente aufweist. Diese Farbpigmente ändern jeweils bei Überschreiten einer bestimmten Grenztemperalur ihre Farbe, sodass die Farbverteilung auf der Folie die maximal erreichte Oberflächentemperatur während des Betriebs des Computers anzeigt. Die Farbstoffträger sind irreversibel verfärbt, sodass sie auch bei einer Abkühlung nicht wieder die ursprüngliche Farbe annehmen.

Das Aufbringen einer derartigen Folie erleichtert es somit, Regionen 33 einer integrierten Schaltung zu erkennen, in denen zumindest kurzzeitig bestimmte Grenztemperaturen überschritten wurden. Da erhöhte Temperaturen die Arbeitsweise einer integrierten Schaltung beeinträchtigen können, kann durch einen geänderten Schaltungsaufbau oder sekundäre Maßnahmen wie beispielsweise Kühlrippen einer derartigen Temperaturerhöhung entgegengewirkt werden.

Eine Temperaturmesssprühschicht kann auch auf die gesamte Platine 30 aufgesprüht werden, um die Bereiche maximaler Temperaturentwicklung während des Betriebes eines Computers zu ermitteln und dieser Temperaturentwicklung gegebenenfalls mit Hilfe eines optimal positionierten Gebläses entgegenzuwirken.

Das Verfahren ist analog auch beispielsweise für feinmechanische Getriebe zu verwenden, um Stellen erhöhter Reibung zu ermitteln, die ebenfalls durch Farbwechsel auf einem aufgesprühten Farbfilm erkannt werden können.

## Patentansprüche

1. Temperaturmesseinrichtung mit einem z. B. auf die Haut eines Menschen oder Tieres aufbringbaren Flächenelement (2), **dadurch gekennzeichnet, dass** das Flächenelement (2) einen Farbstoffträger enthält, der entweder Überschreiten oder bei Unterschreiten einer Grehztemperatur seine Farbe irreversibel ändert.

2. Temperaturmesseinrichtung nach Anspruch 1, ***dadurch gekennzeichnet, dass*** das Flächenelement ein Schriftfeld ist.

3. Temperaturmesseinrichtung nach einem der vorhergehenden AnSprüche, **dadurch gekennzeichnet, dass** der Farbstoffträger bei einer Temperatur zwischen 35 °C und 38 °C seine Farbe ändert.

4. Temperaturmesseinrichtung nach einem der vorhergehenden Ansprüche **dadurch gekennzeichnet, dass** der Farbstoffträger mehrere verschiedene Farbstoffpartikel aufweist, die bei Überschreiten verschiedener Grenztemperaturen jeweils ihre Farbe ändern.

5. Temperaturmesseinrichtung nach einem der vorherigen Ansprüche, **dadurch gekennzeichnet, dass** das Flächenelement (2) eine Farbschicht ist.

6. Temperaturmesseinrichtung nach einem der Ansprüche 1-4, **dadurch gekennzeichnet, dass** das Flächenelement (2) eine aufgesprühte Farbe ist.

7. Temperaturmesseinrichtung nach einem der Ansprüche 1-4, **dadurch gekennzeichnet, dass** das Flächenelement (2) abwaschbar ist.

8. Temperaturmesseinrichtung nach einem der Ansprüche 1-4, **dadurch gekennzeichnet, dass** das Flächenelement eine hautverträgliche Creme oder eine Paste ist.

9. Ternperaturmesseinrichtung nach einem der Ansprüche 1-4, **dadurch gekennzeichnet, dass** das Flächenelement (2) ein entfembarer Farbfilm ist.

10. Temperaturmesseinrichtung nach einem der vorherigen Ansprüche, **dadurch gekennzeichnet, dass** das Flächenelement (10) ein der Form der Fläche entsprechend geformtes oder formbares Element ist.

11. Temperaturmesseinrichtung nach einem der vorherigen Ansprüche, **dadurch gekennzeichnet, dass** das Flächenelement (10) Teil eines Büstenhalters ist.

12. Elektronisches Bauteil mit einer Temperaturmesseinrichtung nach einem der Ansprüche 1 bis 11.

13. Lebensmittel verpackung mit einer Temperaturmesseinrichtung nach einem der Ansprüche 1 bis 11.

14. Verpackung für ein Medikament oder Kosmetik mit einer Temperaturmesseinrichtung nach einem der Ansprüche 1 bis 11.

15. Überdruckgasflasche mit einer Temperaturmesseinrichtung nach einem der Ansprüche 1 bis 11.

## Claims

1. A temperature measuring device with a surface element (2) which can for example be applied to the skin of a person or an animal, **characterised in that** the surface element (2) comprises a colourant carrier which if a boundary temperature is either exceeded or not reached, irreversibly changes its colour.

2. The temperature measuring device according to claim 1, **characterised in that** the surface element is a text field.

3. The temperature measuring device according to one of the preceding claims, **characterised in that** the colourant carrier changes its colour at a temperature between 35 °C and 38 °C.

4. The temperature measuring device according to any one of the preceding claims, **characterised in that** the colourant carrier comprises several different colourant particles which change their colour when different boundary temperatures are exceeded.

5. The temperature measuring device according to any one of the preceding claims, **characterised in that** the surface element (2) is a colour layer.

6. The temperature measuring device according to any one of claims 1 - 4, **characterised in that** the surface element (2) is a sprayed-on colour.

7. The temperature measuring device according to any one of claims 1 - 4, **characterised in that** the surface element (2) is washable.

8. The temperature measuring device according to any one of claims 1 - 4, **characterised in that** the surface element is a creme or paste which is gentle on the skin.

9. The temperature measuring device according to any one of claims 1 - 4, **characterised in that** the surface element (2) is a removable colour film.

10. The temperature measuring device according to any one of the preceding claims, **characterised in that** the surface element (10) is an element which is formed or formable according to the form of the surface.

11. The temperature measuring device according to any one of the preceding claims, **characterised in that** the surface element (10) forms part of a bra.

12. An electronic component comprising a temperature measuring device according to any one of claims 1 to 11.

13. A packaging for foodstuffs comprising a temperature measuring device according to any one of claims 1 to 11.

14. The packaging for medicaments or cosmetics comprising a temperature measuring device according to any one of claims 1 to 11.

15. A pressurised gas cylinder comprising a temperature measuring device according to any one of claims 1 to 11.

## Revendications

1. Dispositif de mesure de température comportant un élément de surface (2) pouvant être appliqué par exemple sur la peau d'un être humain ou d'un animal, **caractérisé en ce que** l'élément de surface (2) contient un support de colorant qui change irréversiblement de couleur soit en cas de dépassement, soit en cas de non-atteinte, d'une température limite.

2. Dispositif de mesure de température selon la revendication 1, **caractérisé en ce que** l'élément de surface est un champ d'écriture.

3. Dispositif de mesure de température selon une des revendications précédentes, **caractérisé en ce que** le support de colorant change de couleur à une température située entre 35 °C et 38 °C.

4. Dispositif de mesure de température selon une des revendications précédentes, **caractérisé en ce que** le support de colorant présente différentes particules de colorant qui changent respectivement de couleur en cas de dépassement de différentes températures limites.

5. Dispositif de mesure de température selon une des revendications précédentes, **caractérisé en ce que** l'élément de surface (2) est une couche colorée.

6. Dispositif de mesure de température selon une des revendications 1 à 4, **caractérisé en ce que** l'élément de surface (2) est une couleur pulvérisée.

7. Dispositif de mesure de température selon une des revendications 1 à 4, **caractérisé en ce que** l'élément de surface (2) peut être éliminé par lavage.

8. Dispositif de mesure de température selon une des revendications 1 à 4, **caractérisé en ce que** l'élément de surface est une crème tolérée par la peau ou une pâte.

9. Dispositif de mesure de température selon une des revendications 1 à 4, **caractérisé en ce que** l'élément de surface (2) est un film coloré pouvant être retiré.

10. Dispositif de mesure de température selon une des revendications précédentes, **caractérisé en ce que** l'élément de surface (10) est un élément formé ou modelable en fonction de la forme de la surface.

11. Dispositif de mesure de température selon une des revendications précédentes, **caractérisé en ce que** l'élément de surface (10) est une partie d'un soutien-gorge.

12. Composante électronique comportant un dispositif de mesure de température selon une des revendications 1 à 11.

13. Emballage de produit alimentaire comportant un dispositif de mesure de température selon une des revendications 1 à 11.

14. Emballage pour médicament ou cosmétique comportant un dispositif de mesure de température selon une des revendications 1 à 11.

15. Cartouche de gaz en surpression comportant un dispositif de mesure de température selon une des revendications 1 à 11.
